# EUROPEAN PATENT APPLICATION

(11) **EP 1 374 944 A1**
(43) Date of publication of application: **02.01.2004**
(21) Application number: 03011517.4
(22) Date of filing: 21.05.2003
(51) Int. Cl.: A61M 37/00, A61N 1/32

(54) **Optimization of transcutaneous permeation of compounds**

(30) Priority: 18.06.2002 US 173585
(71) Applicant: GIAMMARUSTI, PEDRO, Santana de Parnaiba-SP-06543-055 (BR)
(72) Inventor: GIAMMARUSTI, PEDRO, Santana de Parnaiba-SP-06543-055 (BR)
(74) Representative: Isern Jara, Nuria

(57) **Abstract**

A non-invasive method of enhancing the permeability of the skin to a biologically active permeant or compound is described utilizing a combination of sonophoresis and chemical enhancers. The previous preparation of the skin using an ultrasonically generated mechanical skin scrubbing action is also described. Synergism brought simultaneously applying iontophoresis, electroporation, mechanical vibrations and magnetophoresis is used to optimize the transcutaneous active permeation of compounds, considerably lowering the time of treatment. The method is intended also for, among others, the non-invasive painless treatment of cellulitis, localized fat, stretch marks and flaccid skin.

## Description

### BACKGROUND OF THE INVENTION

This invention relates generally to the field of compound delivery for obtaining both local and systemic results. More particularly, it relates to a non-invasive method of compound delivery through the epidermis by means of increasing the permeability of the skin through the mild abrasion of the skin via ultrasound mechanical vibrations, chemical enhancers and sonophoresis and the synergetic simultaneous use of iontophoresis, electroporation, mechanical vibrations and magnetophoresis for optimizing transcutaneous compound delivery into the body.

The human skin has barrier properties and stratum corneum is mostly responsible for them, thus it is exactly the stratum corneum, the outer horny layer of the skin , that imposes the greatest barrier to transcutaneous flux of compounds into the body.

The stratum corneum hasn't constant thickness, since it depends on each particular area , being thinner in areas subject to folds and much thicker in the hands palms and feet soles, is a very resistant waterproof membrane that both protects the body from invasion by exterior substances and the outward migration of fluids and dissolved molecules and creates a mechanical and biological shield between the environment and the interior of the body. The stratum corneum is continuously renewed by shedding of dead cells during desquamation and the formation of new corneum cells by the keratinization process.

Considering the permeation of compounds with non-charged molecules into the skin the flux of said compound across the epidermis is controlled by Fick's First Law which states that this flux depends on the diffusion coefficient and on the gradient of concentration of the compound . One important issue to be remembered is that the diffusion coefficient is strongly dependent on the degree of hydration of the skin, significantly increasing with it.

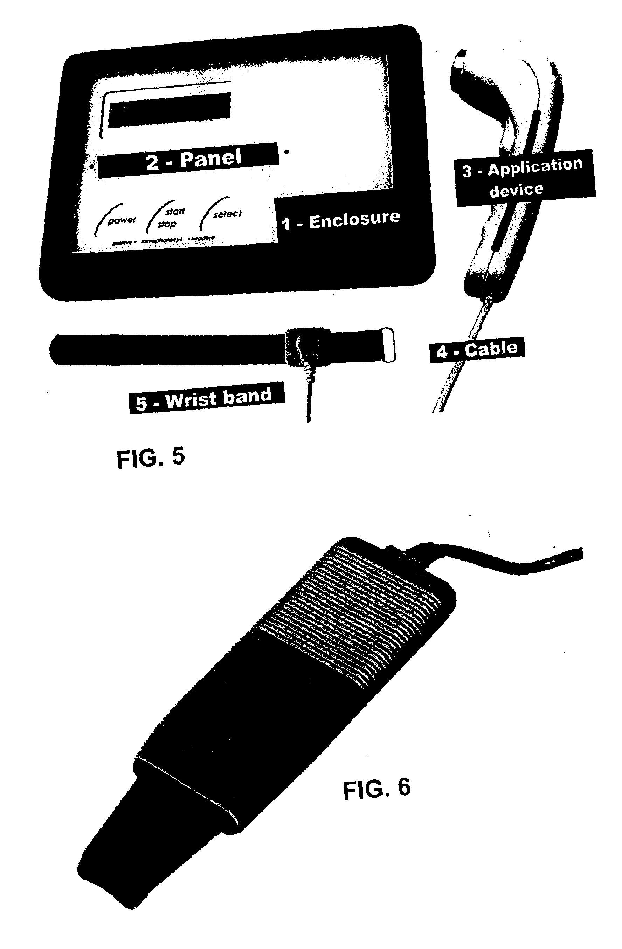

One of the physical ways available for the enhancement of skin permeability is the use of a mild mechanical abrasion (epidermal abrasion) of the wetted surface of the skin with a special ultrasonic transducer roughly having the shape of a spatula with a slightly bent tip (FIG.6 ) ; a chemical peel product may optionally be associated.

First layers of keratinized dead cells are easily removed when this device is gently moved forward some few times on the surface of the stratum corneum; its use allows the possibility of achieving a significant increase of the degree of the hydration of the skin and a decrease in permeation barrier (Novel mechanisms and devices to enable successful transdermal drug delivery - B.W. Barry - European Journal of Pharmaceutical Sciences 14 (2001) 101-114)

Another possible way of enhancing the flux of compounds into the body is through the so-called penetration or chemical enhancers, which increase the coefficient of diffusion of the stratum corneum and may be, associated with sonophoresis, that is, ultrasound energy.

From the physical standpoint ultrasound waves have been defined as mechanical pressure waves with frequencies above 20 KHz, H. Lutz et al., Manual Of Ultrasound 3-12 (1984), are generated by either natural or synthetic materials that show the so-called piezoelectric property, meaning that these materials both generate an electric field when mechanically stressed (the so-called direct piezoelectric effect) and also generate a mechanical force when an electric field is conveniently applied to them (the so-called inverse piezoelectric effect).

These properties have been first established by Pierre and Jacques Curie who have observed their occurrence in natural materials like the Rochelle salt; however in our days synthetic piezoceramic materials are preferred instead due to their more stable properties since they are not hygroscopic and also to the possibility of being manufactured in any shape, allowing a lot of different applications in several areas.

Ultrasound has also been used to enhance permeability of the skin and synthetic membranes to compounds and other molecules and its use to increase the permeability of the skin to compound molecules has been called sonophoresis or phonophoresis meaning transportation through sound like waves.

U.S. Pat. No. 4,309,989 to Fahim describes a method of topically applying an effective medication in an emulsion-coupling agent by ultrasound. More particularly, a method of treating a skin condition by applying a medication in an emulsion-coupling agent and massaging it into the affected area with ultrasonic vibrations thereby causing the medication to penetrate into the skin. Specifically, a method and composition for the treatment of Herpes Simplex Type 1 and Type 2 lesions. Also specifically, a method and composition for the treatment of demidox mites. U.S. Pat. No. 4,372,296 to Fahim similarly describes treatment of acnes by topical application of zinc sulphate and ascorbic acid in a coupling agent.

U.S. Pat. No. 4,767,402 to Kost et al. discloses a method using ultrasound to enhance permeation of molecules through the skin and into the blood stream, at a controlled rate. Depending on the compound being infused through the skin, the rate of permeation is increased as well as the efficiency of transfer. Drugs which may not be effective under other conditions, for example, due to degradation within the gastrointestinal tract, can be effectively conveyed transdermally into the circulatory system by means of ultrasound. Ultrasound is used in the frequency range of between 20 KHz and 10 MHz, the intensity ranging between 0 and 3 W/cm.sup.2. The molecules are either incorporated in a coupling agent or, alternatively, applied through a transdermal patch.

U.S. Pat. No. 4,780,212 to Kost et al. teaches use time, intensity, and frequency control to regulate the permeability of molecules through polymer and biological membranes. Further, the choice of solvents and media containing the molecules also affects permeation of the molecules through the membranes.

U.S. Pat. No. 4,821,740 to Tachibana et al. discloses an endermic application kit for external medicines, which comprises a drug-containing layer as provided near an ultrasonic oscillator. The kit includes a cylindrical fixed-type or portable-type and a flat regular-type or adhesive-type, and the adhesive-type may be flexible and elastic. The drug absorption is ensured by the action of the ultrasonic waves from the oscillator and the drug release can be controlled by varying the ultrasonic wave output from the oscillator.

U.S. Pat. No. 5,007,438 to Tachibana et al. is described an application kit in which a layer of medication and an ultrasound transducer are disposed within an enclosure. The transducer may be battery powered. Ultrasound causes the medication to move from the device to the skin and then the ultrasound energy can be varied to control the rate of administration through the skin.

U.S. Pat. No. 5,115,805 to Bommannan et al. discloses a method for enhancing the permeability of the skin or other biological membrane to a material such as a drug is disclosed. In the method, the drug is delivered in conjunction with ultrasound having a frequency of above about 10 MHz. The method may also be used in conjunction with chemical permeation enhancers and/or with iontophoresis. It is informed but not shown that chemical penetration enhancers and/or iontophoresis could be used in connection with the ultrasound treatment.

U.S. Pat. No. 5,444,611 to Eppstein et al. describes a method of enhancing the permeability of the skin or mucosa to a biologically active permeant or drug utilizing ultrasound or ultrasound plus a chemical enhancer. Ultrasound can be modulated and frequency modulated ultrasound from high to low frequency can develop a local pressure gradient directed into the body. The method is also useful as a means for application of a tattoo by noninvasively delivering a pigment through the skin surface. Due to the completeness of that disclosure, the information and terminology utilized therein are incorporated herein by reference.

U.S. Pat. No. 6,041,253 to Kost et al. describes a method for transdermal transport of molecules during sonophoresis (delivery or extraction) further enhanced by application of an electric field, for example electroporation of iontophoresis. This method provides higher drug transdermal fluxes, allows rapid control of transdermal fluxes, and allows drug delivery or analyte extraction at lower ultrasound intensities than when ultrasound is applied in the absence of an electric field. Due to the completeness of that disclosure, the information and terminology utilized therein are incorporated herein by reference.

U.S. Pat. No. 6,234,990 to Rowe et al. discloses methods and devices for application of ultrasound to a small area of skin for enhancing trasdermal transport. An ultrasound beam having a first focal diameter is channelled into a beam having a second, smaller diameter without substantial loss of energy. A two-step noninvasive method involves application of ultrasound to increase skin permeability and removal of ultrasound followed by transdermal transport that can be further enhanced using a physical enhancer. Due to the completeness of that disclosure, the information and terminology utilized therein are incorporated herein by reference.

Many other references teach use of ultrasound to deliver drugs through the skin, including Do Levy et al., 83 J. Clin. Invest. 2074 (1989); P. Tyle & P. Agrawala, 6 Pharmaceutical Res. 355 (1989); H. Benson et al., 8 Pharmaceutical Res. 1991); D. Bommannan et al., 9 Pharmaceutical Res. 559 (1992); K. Tachibana, 9 Pharmaceutical Res. 952 (1992);N.N. Byl,Physical Therapy , Volume 75 , Number 6, (1995).

Many authors report the success of application of sonophoresis, J. Griffin et al., 47 Phys. Ther. 594 (1967); J. Davick et al., 68 Phys. Ther. 1672 (1988); D. Bommannan et al., 9 Pharm. Res. 559 (1992) . H. Pratzel et al., 13 J. Rheumatol. 1122 (1986); H. Benzon et al., 69 Phys. Ther. 113 (1989)

More recent studies of sonophoresis show that application of ultrasound at therapeutic frequencies of about 1 MHz induces cavitation, that is growth and oscillations of air pockets present in the keratinocytes of the stratum corneum disorganizing the stratum corneum lipid bilayers thereby enhancing transcutaneous transport.

According to U.S. Pat. No. 6,041,25 to Kost, the effect of cavitation on the enhancement of the permeability of the skin is even better when low frequency ultrasound in the range of 20 KHz is used

This means that permeation using only chemical enhancers and sonophoresis can result in an effective process, besides this other physical principles can be added to improve the process in order to create a method for actively and safely enhancing the flux rate of compounds into the skin to a greater extent than can be achieved without their use .

From now on we will be concerned with ionic permeation, the transportation of charged particles, then we have to consider the general diffusion equation instead where Fick's First Law must be added of a second term, normally an electric potential gradient term, meaning another driving force created by an electrical field applied between the area under treatment and a referential electrode, the so-called process of iontophoresis.

Iontophoresis involves the topical delivery of either an ionized form of compound or an unionized compound carried with the water flux associated with ion transport, the process being termed electro-osmosis.

An electrical field is created between the area under treatment and a referential electrode usually fixed to the right wrist of the individual, normally consisting of a variable electric field with selected properties like amplitude, frequency, wave shape, polarity and duty cycle.

The polarity of the electric field depends on the chemicals to be delivered into the skin, therefore it must be accordingly indicated by the manufacturer of Said chemicals and selected by the user.

Low and medium frequency periodic mechanical vibrations created for example by rotating unbalanced masses applied to the skin surface create mechanical pressure waves that establish a pumping action, forcing the compounds into the skin, enhancing the permeation process.

Optimum results are obtained using time varying vibrations, resulting in several types of periodic complex wave shapes like "saw tooth", "triangle", "on-off" and "staircase" among others, with fundamental frequencies in the range of 1 Hz to 1 KHz , but with several useful low order harmonic terms having amplitudes , frequencies and phases according to their respective expansion using Fourier's series.

Finally , one more physical principle can be used to achieve further enhancement of flux rate : constant or time varying magnetic fields which can induce mechanical forces to moving charged particles in such a way to force them into the skin, the so-called process of magnetophoresis.

It is worthwhile to remember that also some heat is internally generated by sonophoresis and additionally also iontophoresis contributes with Joule's effect originated heat , all them giving some contribution to the increase of temperature of the skin .

The increase of temperature of the skin lowers its electrical impedance (A Mechanistic Study of Ultrasonically-Enhanced Transdermal Drug Delivery - Mitragotri et al. - Journal of Pharmaceutical Sciences - Vol.84, No. 6, June 1995 ), improving the efficiency of the process of iontophoresis, however some prevision must be made to keep this temperature under control within safe limits. Same article reports the increase of the permeation coefficient of the skin with the increase of the temperature due to sonophoresis.

Also the local elevation of temperature accelerates lipolysis rate as informed by U.S. Pat. No. 5,507,790 to Weiss.

### OBJECTS AND SUMMARY OF THE INVENTION

An object of the present invention is to provide a method for fast active transcutaneous permeation of compounds through the human skin targeting obtaining either local or systemic results allowing, among others, the non-invasive painless treatment of cellulitis, localized fat, stretch marks and flaccid skin .

Another object of the invention is to provide a method for the active transcutaneous permeation of compounds in a non invasive basis, allowing treatments with minimal occurrence of undesirable collateral effects.

A further object of the invention is to minimize the time of treatment through the synergetic simultaneous use of both chemical enhancers and several physical principles.

These and other objects may be accomplished by creating a mild mechanical abrasion of the skin and applying to the skin surface permeation enhancers and compounds simultaneously with physical permeability enhancers such as sonophoresis with modulated or non-modulated ultrasound, continuous or pulsed, iontophoresis, electroporation, mechanical vibrations and magnetophoresis.

Specially designed equipment and application devices allowing the application of this method will be described herein.

Ultrasound energy also may also open up diffusional pathways in the stratum corneum, causing an increase in the permeability of that layer and causing frictional heat to be generated in deeper tissues, increasing the activity of both lymph and blood circulation, as well as of metabolic processes.

Due to the complete synergism and complementarity of these physical principles, their combined actions lead us to fast treatments when associated with ultrasound coupling products, chemical permeants, allowing compounds to be efficiently permeated through the skin, since both ultrasound, iontophoresis, electroporation, mechanical vibrations and magnetophoresis force chemical enhancers and compounds into the stratum corneum, thereby reducing the lag time associated with the non-enhanced (passive) diffusion process.

### BRIEF DESCRIPTION OF THE DRAWINGS

FIG. 1 shows the distribution of energy fields produced by an ultrasound transducer and division into near field (Fresnel field ) and far field ( Fraunhofer field ).

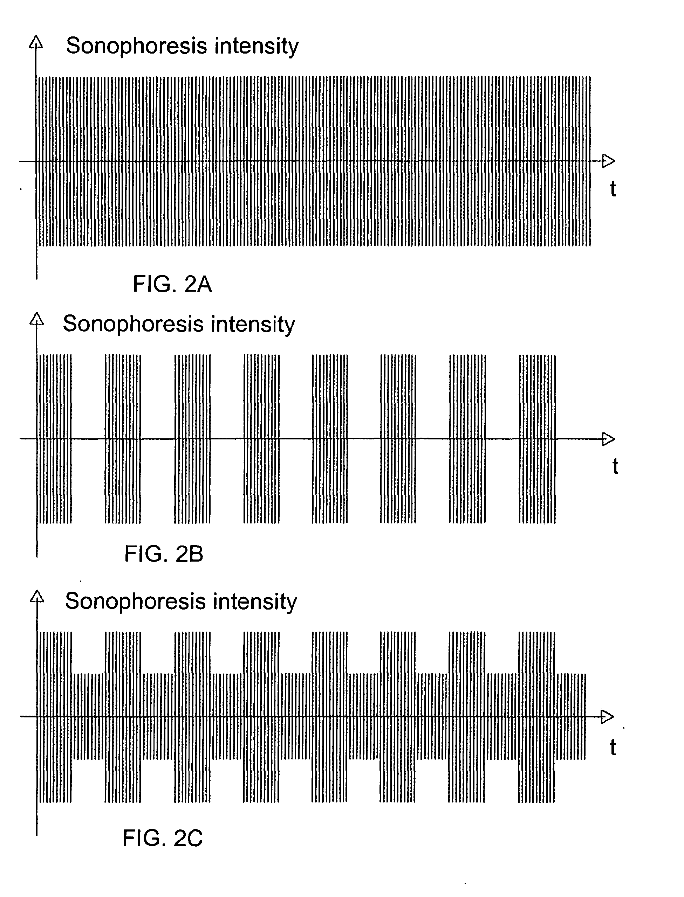

FIG. 2 A shows an example of continuous non-modulated ultrasound wave.
FIG. 2 B shows an example of pulsed ultrasound wave.
FIG. 2 C shows an example of amplitude modulated ultrasound wave.
FIG. 3 A shows an example of non-modulated electrical field according to the present invention.

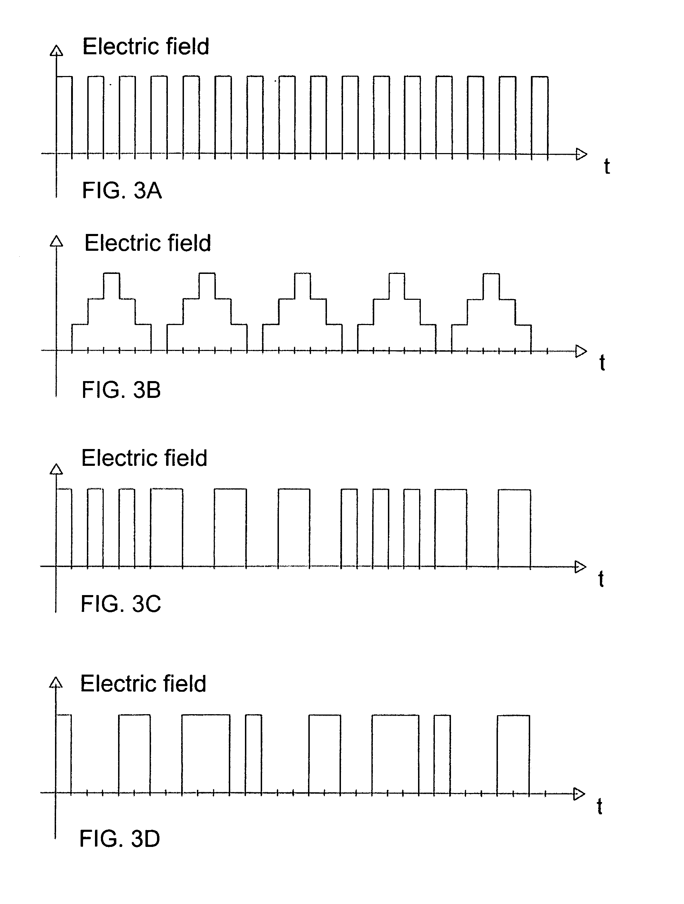

FIG. 3 B shows an example of amplitude modulated electrical field according to the present invention.
FIG. 3 C shows an example of frequency modulated electrical field according to the present invention.
FIG. 3 D shows an example of duty cycle modulated electric field according to the present invention.

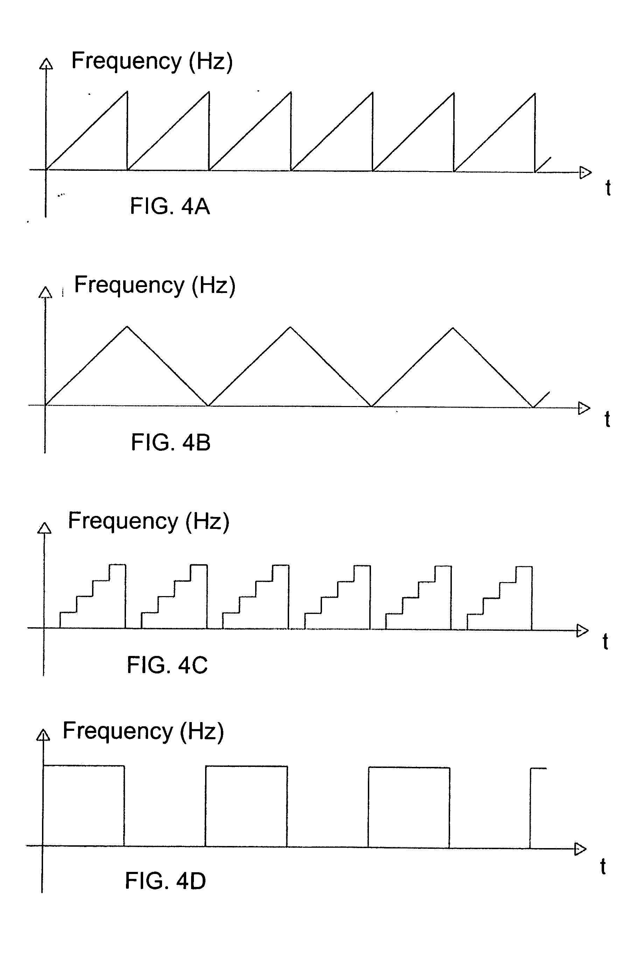

FIG. 4 A shows an example of "saw tooth" frequency wave shape for mechanical vibrations according to the present invention.
FIG. 4 B shows an example of "triangle" frequency wave shape for mechanical vibrations according to the present invention.
FIG. 4 C shows an example of "staircase" frequency wave shape for mechanical vibrations according to the present invention.
FIG. 4 D shows an example of "on-off" frequency wave shape for mechanical vibrations according to the present invention.
FIG. 5 - Perspective view of an experimental equipment and application device, sonophoresis in the therapeutic ultrasound frequency range (~1MHz).
FIG. 6 - Perspective view of an experimental application device suitable for mild mechanical abrasion of the skin surface and simultaneous application of sonophoresis and iontophoresis, sonophoresis in the low frequency ultrasound range (∼28 KHz).

### DETAILED DESCRIPTION OF THE INVENTION

Although the present invention, as described herein, presents the best approach presently known for enhancing the permeability of membranes using ultrasound and enhancing the transcutaneous flux rate of a compound through a biological membrane through the use of chemical permants and sonophoresis, iontophoresis, electroporation, mechanical vibrations and magnetophoreis, it is to be understood that this invention is not limited to the particular process steps and materials disclosed herein as such process steps and materials may vary.

It is also to be understood that the terminology used herein is not intended to be limiting since the scope of the present invention will be limited only by the appended claims and their equivalents.

This invention is intended to establish an optimized mode of delivery of agents or permeants which exist in the state of the art or which may later be established as active agents and which are suitable for delivery by the present invention, including compounds normally delivered into the body, through body surfaces and membranes, including skin.

As used herein, "transcutaneous" has the same meaning of transdermal or percutaneous.

As used herein, a "biological membrane" is intended to mean also the human skin.

As used herein, "individual" refers to a human, to which the present invention may be applied.

As used herein, "transcutaneous flux rate" is the rate of passage of any compound, pharmacologically active agent, through the skin of an individual.

As used herein, "low frequency ultrasound" means ultrasound waves with frequencies below or equal to 1 MHz.

As used herein, "therapeutic ultrasound" means ultrasound waves with frequencies above 1 MHz.

As used herein, "non-invasive" means not requiring the entry of a needle, catheter, or other invasive medical instrument into any part of the body including its natural orifices like mouth, nose, ears, anus, urethra and vagina.

Firstly speaking of permeation of non charged particles, Fick's First Law states that the flux of a compound across the skin can be varied by changing either the diffusion coefficient or the driving force, that is the gradient of concentration. In a simplified way this means that if the gradient of concentration is constant, then the transcutaneous flux rate can only be enhanced by improving the diffusion coefficient. This can be achieved by the use of so-called penetration or chemical enhancers associated with sonophoresis.

There are two primary categories of components where chemical enhancers are comprised of, that is, cell-envelope disordering compounds and solvents or binary systems containing both cell-envelope disordering compounds and solvents, where the first are well known as being useful in topical pharmaceutical preparations therefore any cell envelope disordering compound is useful for purposes of this invention.

Cell-envelope disordering compounds are thought to assist in skin penetration by disordering the lipid structure of the stratum corneum cell-envelopes; solvents include water; diols, mono-alcohols, DMSO and others.

European Patent Application 43,738 presents the use of selected diols as solvents along with a broad category of cell-envelope disordering compounds for delivery of lipophilic pharmacologically-active compounds. Because of the detail in disclosing the cell-envelope disordering compounds and the diols, this disclosure of European Patent Application 43,738 is incorporated herein by reference.

Other chemical enhancers, not necessarily associated with binary systems, include DMSO or aqueous solutions of DMSO such as taught in Herschler, U.S. Pat. No. 3,551,554; Herschler, U.S. Pat. No. 3,711,602; and Herschler, U.S. Pat. No. 3,711,606, and the azones (n-substituted-alkyl-azacycloalkyl-2-ones) such as noted in Cooper, U.S. Pat. No. 4,557,943.

Some chemical enhancer systems may show negative collateral effects such as toxicity and skin irritation. U.S. Pat. No. 4,855,298 discloses compositions for reducing skin irritation having an amount of glycerine sufficient to provide an anti-irritating effect.

Since this invention is not drawn to the use of chemical enhancers per se it is believed that all chemical enhancers useful in the delivery of compounds through the skin may be associated with sonophoresis, iontophoresis, mechanical vibrations and magnetophoresis in further enhancing the delivery of permeants and compounds through the skin surface.

Permeation through the stratum corneum can occur either by intracellular, intercellular or transappendageal penetration, in this case specially through the aqueous pathway of the sweat glands. The property shown by the ultrasound of enhancing the permeability of the stratum corneum and, consequently, increasing transcutaneous flux rate is thought to derive from thermal and mechanical alteration of biological tissues.

The physical properties of ultrasound waves that can be changed either to control or improve penetration include frequency and intensity along with time of application. Other factors are also important, for example the composition and structure of the membrane through which molecules are to be transported, the physical and chemical characteristics of the medium in which the molecules are suspended, and the nature of the molecules themselves.

The exposure may be either continuous or pulsed to reduce excessive heating of biological membranes, when upper average values of usual intensities in the range of 0.01-2.5 W/cm.sup.2 are used; Selection is made in such a way to intensity be high enough to achieve the desired results as well as low enough to avoid significant increase of skin temperature. However, using our experimental equipment and application devices intensities between 0.2 and 1.5 W/cm.sup.2 have shown to give good results when the process is associated with simultaneous application of iontophoresis.

Ultrasound frequencies varied from 20 kHz to 10 MHz, preferably 20 KHz to 3 MHz taking into account that the practical depth of penetration of ultrasonic energy into living soft tissue due to attenuation is inversely proportional to some power of the frequency; high frequencies have been suggested to improve drug penetration through the skin by concentrating their effect in the stratum corneum but frequencies between 1 to 3 MHz show a better overall efficiency since they create some deeper internal heat producing a temperature rise that speeds up metabolic processes in the area under treatment as well as lowering the electrical impedance of the skin, improving the iontophoreis process.

No significant cavitational effects is observed in fluids at ultrasound frequencies greater than 2.5 MHz, due to the fact that these cavitational effects vary inversely with ultrasound frequency [Gaertner, W., Frequency dependence of ultrasonic cavitation, J. Acoust. Soc. Am., 26:977-80 (1984)], therefore 2.5 MHz is considered a reasonable estimate of the upper frequency limit for the occurrence of cavitation in fluids at therapeutic ultrasound intensities.

On the other hand, cavitation originated using low frequency ultrasound, in the range of 20 KHz has shown to be very effective in the enhancement of the skin permeabilty ( U.S. Pat. No. 6,041,253 to Kost ), allowing the permeation of higher molecular weight molecules, well above 600 D.

Hence, since cavitation plays an important role in transcutaneous permeation, the synergistic effect of sonophoresis and iontophoresis shall be nearly absent with frequencies higher than 2.5 MHz.

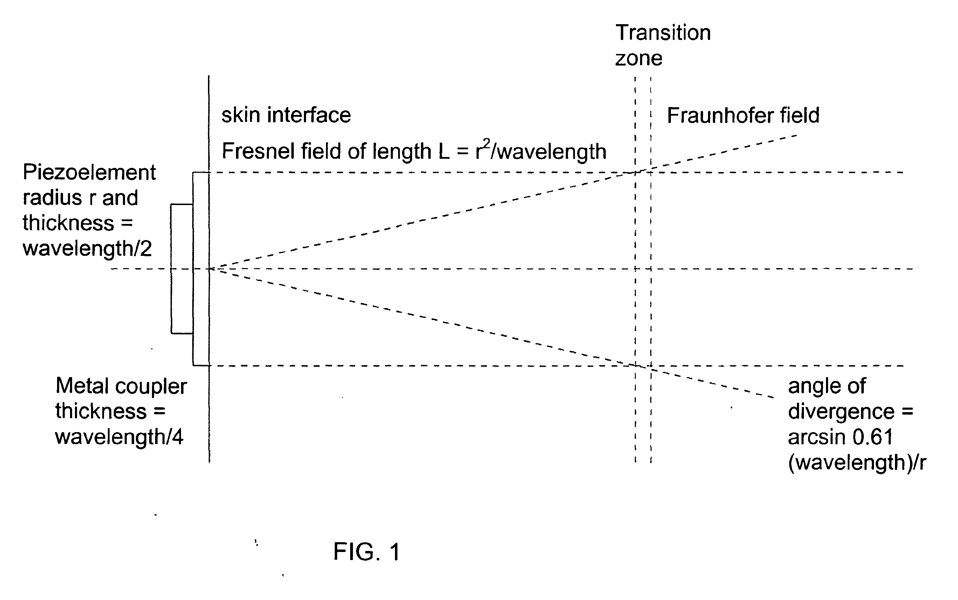

When ultrasound energy is applied into the body using for example a circular plane metallic transducer two fields are created, the near field, known as Fresnel field and the far field, known as Fraunhofer field as shown in FIG. 1.

In Fresnel field ultrasound energy radiated from different parts of the element travels as spherical waves that interfere both constructively and destructively; thus there are zones of maxima and minima of mechanical pressure along and across the beam. This field is characterized by a length which depends on the radius of the radiant surface and the wavelength of the ultrasound in the medium in front of it, i.e., the skin and soft tissues beneath it.

Therefore the ultrasound energy distribution pattern shows a large number of closely spaced local mechanical pressure peaks and nulls. The energy is "channelled" into the skin in a structure having parallel "walls" orthogonal to the plane of the transducer face.

In Fraunhofer field the ultrasound beam diverges in such a way which also depends on the radius of the radiant surface and the wavelength of the ultrasound in the medium, usually soft tissues, meaning that in Fraunhofer field the energy is spreaded in a conic distribution .

The interface of the piezoelectric transducer with the individual is reflective due to the different values of their respective acoustic characteristic impedances and energy is reflected back to the piezoelement. Thus, in order to obtain constructive interference, that is reinforcement of the ultrasound waves, the thickness of the piezoelectric transducer, normally circular shaped, must be one-half wavelength for the frequency used.

In one of the embodiments used, experiments were conducted with an application device having a lead zirconate titanate transducer 2 mm thick , and since the speed of sound for this material is of 4000 m/sec, the frequency which allows maximum energy transfer for such device is of 1 MHz.

By many reasons the individual must be mechanically isolated from the piezoelectric element, and usually this is achieved interposing a plate of material having an intermediate acoustic characteristic impedance between them; in order to maximize the energy transfer, this plate must have a thickness of one quarter wavelength for the frequency being used.

This application device used an aluminium plate for this purpose and since the speed of sound for this material is of 6400 m/s then best results were obtained with a plate 1.6 mm thick.

In order to minimize reflexions of the ultrasonic beam, which depend on the ratio of the acoustic characteristic impedances of the media it is crossing we must avoid any air gap in the interface between the application device and the surface of the skin. Thus a coupling agent, preferably one having a low absorption coefficient of ultrasound energy and being non-staining, non-irritating and slow drying must be topically applied to the skin to efficiently transfer the ultrasonic energy from the ultrasound transducer into the skin.

This way the ultrasound coupling agent can be also formulated along with chemical enhancers and drugs to be permeated, the resulting compounds known as "melanges" .

The above description shows that each particular application device must be operated in a single frequency, otherwise internal acoustic mismatches will cause only partial transfer of energy to the individual, decreasing the efficiency of the process.

Besides this, there will be a considerable overheating of the transducer created by the internal reflected waves, which can negatively affect the mechanical integrity of the transducer, as well as causing a degradation of its piezoelectric properties along the time.

Some different time patterns of peaks and nulls can be obtained with non-modulated ultrasound energy mechanically travelling the transducer back and forth on the surface of the area under treatment, since the results will be quite similar to an "on-off" amplitude modulation, displacing the areas of maxima and minima of pressure along the time .

Application of electric current enhances transcutaneous transport by different mechanisms, for example it provides an additional driving force for the transport of charged molecules across the skin since electrical current paths can be established through the intercellular spaces of the cells of the stratum corneum and second, ionic motion due to application of electric fields may induce convective flows across the skin, referred to as electro-osmosis, an important mechanism in transcutaneous transport of neutral molecules during iontophoresis.

Also and it is thought to have additional paths through the salty sweat glands fluids which show a low electrical impedance to the current flow due to the comparatively low impedance nature of sweat.

Frequencies can range from 5 KHz to 1 MHz, often in the range of 50 KHz to 150 KHz and rectangular voltage with amplitudes from 0 to 15 V or current waves with amplitudes from 0.01 to 1.0 mA/cm.sup.2 with properly selected duty cycles are convenient to achieve good results.

Therefore current waves obtained through electronic generators having high internal impedance are preferable instead since their amplitudes don't depend on fluctuations on the value of skin electrical impedance, allowing safer and more reliable treatments.

Amplitudes shall be kept small enough not to originate either tissue electrical stimulation or excessive heat due to Joule effect. Good results have been obtained with values about 0.5 mA/cm.sup.2 or even lower than this, due to the synergistic simultaneous application of sonophoresis.

U.S.Pat. No. 5,507,790 to Weiss discloses that with the use of iontophoresis the penetration of compounds through the skin can be as deep as 3 or 4 mm.

Mechanical vibrations create pressure gradients which enhance the physical movement of compounds into the skin, improve both lymph and blood circulation in the area as well as create physical stimuli which have a physiological response from the individual, since pressure sensitive nervous terminations of tissues in the area being treated are stimulated and respond to these stimuli increasing the speed of some metabolic processes.

These pressure waves are inertially created through an unbalanced rotating mass fixed to the shaft of a direct current (DC) micro motor having its speed controlled by a pulse width modulation technique ( PWM ), allowing time varying speeds to be synthesized.

In our experiences several different frequency wave shapes have been used, i.e., saw tooth, triangle, on-off, staircase, constant low speed, constant high speed, periodic switching from low to high speed as well as any combinations of them; all time varying frequency wave shapes have given better results, probably due to time varying pressure gradients created as well as the property of the individual to have better perception and responses to changes; of course other wave shapes can be used with the present invention.

Magnetophoresis in based on the law of Electromagnetism which states that when moving charged particles cross a magnetic field they are subject to the action of forces; thus charged molecules of chemicals being permeated can further have a driving force applied to them by means of convenient magnetic fields having such magnitude, direction and polarity in order to enhance the process of transcutaneous permeation.

These magnetic fields may be created by either the circulation of electric currents through specially developed coils placed inside the application device and through permanent magnets.

### PRACTICAL EMBODIMENTS

In order to have a better understanding of both the equipment and the application devices developed for the purposes of this invention, they will be described making reference to FIG. 5 where a perspective view is shown and FIG.6 where the skin scrubbing application device is shown.

### FIRST EMBODIMENT

The first embodiment allows the use of therapeutic ultrasound frequencies, in this case, of 1 MHz.

According to FIG. 5, in this embodiment the experimental equipment consists of a main unit comprised of an enclosure (#1), which can be metallic, plastic or using any other similar materials, which encloses all electronic circuitry needed for its operation.

At the front part of this main unit (#1) there is a panel (#2) with several controls, displays and signalling devices in order to allow an interfacing with the user as friendly as possible .

The equipment also has a manual application device (#3) made of plastic, metal and/or similar materials connected to the main unit (#1) by an electrical cable (#4) using an appropriate connector.

A conductive wrist band (#5) is used to connect the main unit to the individual under treatment through an helicoidal electrical cable .

The application device has an internal ultrasound transducer for the generation of 1 MHz ultrasound waves for sonophoresis, mechanically coupled to a 35 mm metallic circular plate, designed to achieve best enhancement in the skin permeability as described herein.

Either iontophoresis and electroporation may be obtained through the application of an electric variable field between the metallic surface of the application device and the skin, the electric path being closed through the conductive wrist band attached to the wrist of the individual under treatment.

A switch was included in order to reverse the polarity of the electric field, according to the pH of the melange being used; this switching action can also be achieved electronically. Amplitude, frequency and duty cycle of a rectangular current wave have were modulated targeting best results; also pulsed rectangular waves have been used for the same purpose.

Amplitudes of currents for iontophoresis have been varied in the range of 0.1 to about 1 mA/cm.sup.2 with better results obtained for currents higher than 0.5 mA/cm.sup.2.

Low frequency mechanical vibrations are generated internally to the application device by means of an internal unbalanced rotating mass with speed controlled through pulse width modulating the DC voltage applied to the driving electric DC micro motor. Frequencies of 1 Hz to 200 Hz were used with several speed wave shapes as previously described.

Both constant and variable magnitude magnetic field are generated by electrical currents passing through a special coil internal to the application device .

Since also some spatially distributed internal heat is generated by sonophoresis and also conductive heating is produced by Joule effect at the face of the metallic plate of the application device, temperature of the application device is continuously sensed through a thermal sensor allowing this temperature to be always kept under 41.degree.C , using a micro controller and associate electronic circuitry.

This way in normal use some drops of the melange to be permeated into the skin are topically dispensed and them the application device is moved in circular patterns over the skin covering the area under treatment till the complete permeation of the melange is achieved.

### SECOND EMBODIMENT

The second embodiment allows either mechanical scrubbing action of the stratum corneum and low frequency sonophoresis, in this case , of 28 KHz.

According to FIG. 5 and FIG. 6, in this new embodiment the experimental equipment consists of a main unit comprised of an enclosure (#1), which can be metallic, plastic or using any other similar materials, which encloses all electronic circuitry needed for its operation.

At the front part of this main unit (#1) there is a panel (#2) with several controls, displays and signalling devices in order to allow an interfacing with the user as friendly as possible.

The equipment also has a manual application device (#3) made of plastic, metal and/or similar materials connected to the main unit (#1) by an electrical cable (#4) using an appropriate connector .

A conductive wrist band (#5) is used to connect the main unit to the individual under treatment through an helicoidal electrical cable .

The application device has several internal ultrasound transducers for the generation of 28 KHz ultrasound waves useful for either mechanical skin abrasion and sonophoresis. The tip of the application device, with the shape of a spatula is made of stainless steel.

This application device is designed in such way to be used in two different positions:

In the first, its tip is kept with a tilt angle of around 60 degree with respect to the skin surface and this way the mechanical excursion of its tip allows a scrubbing action of the wetted skin as well as generating cavitation of the wetting fluid .

In the second, the metallic surface of the application device is kept flat on the surface of the skin generating cavitation of the lipids of the stratum corneum in order to achieve best enhancement of the skin permeability as described herein .

In this second position either iontophoresis and electroporation may be obtained through the application of an electric variable field between the metallic surface of the application device and the skin, the electric path being closed through the conductive wrist band attached to the wrist of the individual under treatment.

A switch was included in order to reverse the polarity of the electric field, according to the pH of the melange being used; this switching action can also be achieved electronically. Amplitude, frequency and duty cycle of a rectangular current wave have been modulated targeting best results; also pulsed rectangular waves have been used for the same purpose.

Amplitudes of currents for iontophoresis have been varied in the range of 0.1 to about 1 mA/cm.sup.2 with better results obtained for currents equal or higher than 0.5 mA/cm.sup.2.

Since also some spatially distributed internal heat is generated by sonophoresis and also conductive heating is produced by Joule effect at the face of the metallic plate of the application device, temperature of the application device is continuously sensed through a thermal sensor allowing this temperature to be always kept under 41.degree.C, using a micro controller and associate electronic circuitry.

In this embodiment, first the skin must be wetted with a chemical having for example cleansing properties, the application device being used in the first position.

After this mechanical scrubbing action the skin shall optionally be hydrated by an appropriate hydration agent and after this some drops of the melange to be permeated into the skin are topically dispensed and then the application device is placed in the second position and moved in circular patterns over the skin covering the area under treatment till the complete permeation of the melange is achieved.

### EXPERIMENTS

Experiment #1 - Comparative treatment of cellulitis with Classical Mesotherapy and Active Transcutaneous Permeation using embodiment # 1.

In this experiment five volunteer patients showing nodular cellulitis (orange skin) in their thighs were submitted simultaneously to twelve sessions of both processes, all them were applied by an authorized physician, two sessions per week. Area covered in each thigh was of about 200 cm.sup2.

Both process used melanges containing lipolysis activators and improvers of the blood microcirculation and quite similar volumes and concentrations of active principles were used.

Sonophoresis has used therapeutic ultrasound, frequency of 1 MHZ and 1,0 W/cm.sup2, inertial mechanical vibrations have used a ON-OFF wave shape, maximum speed of about 4000 RPM, iontophoresis used a 50 KHz, 50 % duty cycle constant current wave with amplitude of 0.5 mA/cm.sup2., Positive polarity.

The treatment of the right thighs via Classical Mesotherapy has used specific injectable melanges employing discardable syringes and appropriate needles. A number of 80 punctures have been made in each session.

After each session the patients have reported the inconvenience of the pain and remaining marks.

The treatment of the left thighs via Active Transcutaneous Permeation has used topically applied phytotherapic melanges; about 5.0 ml of melange were used in each session and duration of the session was of about 5 minutes. No previous superficial preparation of the skin was used.

After each session the patients have reported the complete absence of any pain and remaining marks.

Patients submitted to Active Transcutaneus Permeation process have been asked not to shower within four hours of each session since the remaining melange continues to permeate through the skin; it is important to observe that skin permeability enhancement stands for some few hours after the use of sonophoresis .

After five sessions the two groups of thighs were visually qualitatively inspected and it was found that the process of Active Transcutaneous Permeation showed similar results when compared to the Classical Mesotherapy, but with a significant difference, a higher skin evenness due to the intrinsic homogeneity of the process of application of melanges.

After twelve sessions the results confirmed the previous ones regarding the higher evenness of the skin treated with the process of Active Transcutaneous Permeation.

Experiment #2 - Comparative treatment of localized fat with Classical Mesotherapy and Active Transcutaneous Permeation using embodiment # 1.

In this experiment three volunteer patients showing localized fat in their abdomens were submitted simultaneously to twelve sessions of both processes, all them were applied by an authorized physician, two sessions per week. Area covered in each abdomen was of about 300 cm.sup2.

Both processes used melanges containing lipolysis activators, phosphodiesterase inhibitors and improvers of the blood microcirculation and quite similar volumes and concentrations of active principles were used.

Sonophoresis has used therapeutic ultrasound, frequency of 1 MHZ and 1,0 W/cm.sup2, inertial mechanical vibrations have used a triangle wave shape, maximum speed of about 3500 RPM, iontophoresis used a 50 KHz, 50 % duty cycle constant current wave with amplitude of 0.5 mA/cm.sup2., positive polarity .

The treatment of the right side of the abdomens via Classical Mesotherapy has used specific injectable melanges utilizing discardable syringes and appropriate needles. A number of about 120 punctures have been made in each session.

After each session the patients have reported the inconvenience of the pain and remaining marks.

The treatment of the left side of the abdomens via Active Transcutaneous Permeation has used topically applied phytotherapic melanges; about 7.5 ml of melange were used in each session and duration of the session was of about 7 minutes. No previous superficial preparation of the skin was used.

After each session the patients have reported the complete absence of any pain and remaining marks.

Patients submitted to Active Transcutaneus Permeation process have been asked not to shower within four hours of each session since the remaining melange continues to permeate through the skin; it is important to observe that skin permeability enhancement stands for some few hours after the use of sonophoresis.

After six sessions the two groups of abdomens were visually qualitatively inspected and it was found that the process of Active Transcutaneous Permeation showed similar results when compared to the Classical Mesotherapy, but with a significant difference, a higher skin evenness due to the intrinsic homogeneity of the process of application of melanges.

After twelve sessions the results confirmed the previous ones regarding the higher evenness of the skin treated with the process of Active Transcutaneous Permeation.

Experiment #3 - Comparative treatment of stretch marks with Classical Mesotherapy and Active Transcutaneous Permeation using embodiment # 2.

In this experiment three volunteer patients showing stretch marks in their waistlines were submitted simultaneously to ten sessions of both processes, all them were applied by an authorized physician, two sessions per week. Area covered in each side was of about 150 cm.sup2.

Both processes used melanges containing lipolysis activators, connective tissue nutrients and improvers of the blood microcirculation with quite similar volumes and concentrations of active principles.

The treatment of the right side of the waistlines via Classical Mesotherapy has used specific injectable melanges utilizing discardable syringes and appropriate needles. A number of about 60 punctures have been made in each session.

After each session the patients have reported the inconvenience of the intense pain and remaining marks.

The treatment of the left side of the waistlines via Active Transcutaneous Permeation has used an initial preparation of the skin utilizing the application device for five minutes with the function of creating a mechanical scrubbing action of the wetted skin; a cleanser cosmetic product was used with this purpose.

After this about 3 ml of melange were used in each session, the application device was placed on the surface of the skin parallel to its surface and duration of the session was of about 4 minutes.

Sonophoresis has used low frequency ultrasound, frequency of 28 KHz and 1.0 W/cm.sup2, iontophoresis used a 50 KHz, 50 % duty cycle constant current wave with amplitude of 0.1 mA/cm.sup2.

After each session the patients have reported the complete absence of any pain and residual marks.

Patients have been asked not to shower within four hours of each session since the remaining melange continues to permeate through the skin; it is important to observe that skin permeability enhancement stands for some few hours after the use of sonophoresis.

After five sessions the two groups of waistlines were visually qualitatively inspected and it was found that the process of Active Transcutaneous Permeation showed similar results when compared to the Classical Mesotherapy, but with a significant difference, a better aspect of the reduced stretch marks due to the intrinsic homogeneity of the process of application of melanges.

After ten sessions the results confirmed the previous ones regarding the better aspect of the reduced stretch marks with the process of Active Transcutaneous Permeation.

### CONCLUSION

Many other results obtained with similar embodiments and processes varying the qualitative and quantitative compositions of melanges, chemical enhancers, wave shapes of inertial mechanic vibrations, their maximum speeds, amplitude and frequency of iontophoresis, volume of melanges permeated through the skin as well as other physical parameters such as time of application in treatments of cellulitis, localized fat, stretch marks and flaccid skin with special melanges were encouraging, showing the validity of both the embodiments, processes and the methods of application used.

The above examples and illustrated embodiments and procedures are but representative of systems which may be employed in the utilization of one or more chemical and/or physical enhancement means for the transcutaneous delivery of permeants and compounds.

The invention is directed to the discovery that the proper use of ultrasound skin abrasion, chemical enhancers and ultrasound associated with the simultaneous use of further physical principles through a single application device as described herein enables the noninvasive transcutaneous delivery of compounds.

However, the invention is not limited only to the specific illustrations since there are numerous enhancer systems some of which may function better than another for delivery of permeants and compounds.

Therefore, the invention is limited in scope only by the mentioned claims and functional equivalents thereof.

### REFERENCES CITED

### U.S. Patent Documents

| | | |
|---|---|---|
| 4372296 | Feb., 1983 | Fahim. |
| 4646725 | Mar., 1987 | Moasser. |
| 4767402 | Aug., 1988 | Kost et al.. |
| 4780212 | Oct., 1988 | Kost et al.. |
| 4821740 | Apr., 1989 | Tachibana et al.. |
| 4953565 | Sep., 1990 | Tachibana et al.. |
| 5007438 | Apr., 1991 | Tachibana et al.. |
| 5016615 | May., 1991 | Driller. |
| 5171215 | Dec., 1992 | Flanagan. |
| 5231975 | Aug., 1993 | Bommannan et al.. |
| 5267985 | Dec., 1993 | Shimada et al.. |
| 5445611 | Aug. , 1995 | Eppstein et al.. |
| 6041253 | Mar. , 2000 | Kost et al.. |
| 6234990 | May , 2000 | Rowe et al.. |

### Other References

Eppstein, D.A. et al., "Applications of Liposome Formulations for Antimicrobial/Antiviral Therapy" Liposomes as Drug Carriers 311, 315 (G. Gregoriadis ed. 1988).

Eppstein, D.A. et al., "Alternative Delivery Systems for Peptides and Proteins as Drugs" 5 CRC Reviews in Therapeutic Drug Carrier Systems 99, 125 (1988).

Loshilov, V.I. et al., "Research of the Technological Process of Ultrasound Treatment of Infected Wounds" (1976).

Ulashik, V.S. et al., Ultrasound Therapy (Minsk, Belarus 1983).

Apfel, R.F., "Possiblity of Microcavitation from Diagnostic Ultrasound," IEEE Trans. Ultrason. Ferroelectrics Freq. Control UFFC-33:139-142 (1986).

Barry, "Mode of Action of Penetration Enhancers in Human Skin," J. Controlled Rel. 6:85-97 (1987).

Burnette, R. R., "Iontophoresis," Transdermal Drug Delivery Developmental Issues and Research Initiatives (Hadgraft and Guv. Editors, Marcel Dekker, 247-291, 1989).

Cleary, Gary W., "Transdermal Controlled Release Systems," Medical Applications of Controlled Release (Langer and Wise, Editors, CRC Press 203-251, 1984).

Clegg and Vaz, "Translational diffusion of proteins and lipids in artificial lipid bilayer membranes. A comparison of experiment with theory," Progress in Protein-Lipid Interactions Watts, ed. (Elsvier, NY 1985) Chapter 5:173-229.

Davis, J.,et al., "Characterization of Recombinant Human Erythropoietin Produced in Chinese Hamster Ovary Cells," Biochemistry 26:2633-2638 (1987).

D'Emanuele, et al., "An Investigation of the Effects of Ultrasound on Degradable Polyanhydride Matrices," Macromolecules 25:511-515 (1992).

Elias, "The Microscopic Structure of the Epidermis and Its Derivatives," Percutaneous Absorption: Mechanisms-Methodology-Drug Delivery (Bronaugh, R. L., Maibach, H., Editors, Marcel Dekker, New York,) 1-12 (1989).

Flynn, G. L., "Mechanism of Percutaneous Absorption from Physiochemical Evidence," Percutaneous Absorption: Mechanisms-Methodology-Drug Delivery (Bronaugh, R.L., Maibach, H., Editors, Marcel Dekker, New York) 27-51 (1989).

Gaertner, W., "Frequency Dependence of Ultrasonic Cavitation," J. Acoust. Soc. Am. 26:977-980 (1954).

Hansch and Leo, "Substitutent Constants for Correlation Analysis in Chemistry and Biology" (1979).

Junginger, et al., "Visualization of Drug Transport Across Human Skin and the Influence of Penetration Enhancers," "Drug Permeation Enhancement" (Hsieh, D.S., Editors, Marcel Dekker, Inc. New York) 59-89 (1994).

Kasting, et al., "Prodrugs for Dermal Delivery: Solubility, Molecular Size, and Functional Group Effects," "Prodrugs: Topical and Ocular Delivery" Sloan, ed. (Marcel Dekker, NY 1992) 117-161.

Kost and Langer, "Ultrasound-Mediated Transdermal Drug Delivery," Topical Drug Bioavailability Bioequivalence and Penetration (Maibach, H. I., Shah, V. P., Editors, Plenum Press, New York) 91-104 (1993).

Kost, et al., "Ultrasound Effect on Transdermal Drug Delivery," (Ben Gurion University Dept. of Chem. Engineering, Beer Sheva Israel) (MIT, Dept. of Appliced Biological Sciences, Cambridge, MA) CRS Aug. 1986.

Levy, et al., "Effect of Ultrasound on Transdermal Drug Delivery to Rats and Guinea Pigs," J. Clin. Invest. 83:2074-2078 (1989).

Liu, et al., "Contransport of Estradiol and Ethanol Through Human Skin In Vitro: Understanding the Permeant/Enhancer Flux Relationship," Pharmaceutical Research 8:938-944 (1991) .

Liu, et al., "Experimental Approach To Elucidate the Mechanism of Ultrasound-Enhanced Polymer Erosion and Release of Incorporated Substances," Macromolecules 25:123-128 (1992).

Machluf and Kost, "Ultrasonically enhanced transdermal drug delivery. Experimental approaches to elucidate the mechanism," J. Biomater. Sci. Polymer Edn. 5:147-156 (1993) .

Mitragotri, et al., "Ultrasound-Mediated Transdermal Protein Delivery," Science 269:850-853 (1995).

Mitragotri, et al., "A Mechanistic Study of Ultrasonically-Enhanced Transdermal Drug Delivery," J. Pharm. Sci. 84:697-706 (1995).

Morimoto, Y., et al., "Prediction of Skin Permeability of Drugs: Comparison of Human and Hairless Rat Skin," J. Pharm. Pharmacol. 44:634-639 (1991).

Newman, J., et al., "Hydrocortisone Phonophoresis," J. Am. Pod. Med. Assoc. 82:432-435 (1992).

Perry, et al., "Perry's Chemical Engineering Handbook" (McGraw-Hill, NY 1984).

Pishko, et al., "Amperometric Glucose Microelectrodes Prepared through Immobilization of Glucose Oxidase in Redox Hydrogels," Anal. Chem. 63:2268-2272 (1991).

Potts and Guy, "Predicting Skin Permeability," Pharm. Res. 9:663-669 (1992).

Prausnitz, et al., "Electroporation of mammalian skin:A mechanism to enhance transdermal drug delivery," Proc. Natl. Acad. Sci. USA 90:10504-10508 (1993).

Quillen, W.S., "Phonophoresis: A Review of the Literature and Technique," Athl. Train. 15:109-110 (1980).

Robinson & Lee, "Influence of Drug Properties on Design," Controlled Drug Delivery 42-43.

Rosell, J., et al., "Skin Impedance From 1 Hz to 1 MHz," IEEE Trans. Biomed. Eng. 35:649-651 (1988).

Skauen, et al., "Phonophoresis," Int. J. Pharm. 20:235-245 (1984) .

Tyle and Agrawala, "Drug Delivery by Phonophoresis," Pharm. Res. 6:355-361 (1989).

Walmsley, "Applications of Ultrasound in Dentistry," Ultrasound in Med. and Biol. 14:7-14 (1988).

Walters, K. A., "Penetration Enhancers and Their Use in Transdermal Therapeutic Systems," Transdermal Drug Delivery: Developmental Issues and Research Initiatives, 197-246 (Hadgraft J., Guy, R.H., Editors, Marcel Dekker, 1989).

Wester and Mailbach, "Animal Models for Percutaneous Absorption," Topical Drug Bioavailability Bioequivalence and Penetration (Shah and Maibach, Editors, Plenum Press, New York) 333-349, (1993).

Williams, et al., "On the non-Gaussian distribution of human skin permeabilities," Int. J. Pharm. 86:69-77 (1992).

Wilschut, et al., "Estimating Skin Permeation, The Validation of Five Mathematical Skin Permeation Models," Chemosphere 30:1275-1296 (1995).

## Claims

1. A non-invasive method for enhancing the transcutaneous flux rate of an active permeant and either ionized form of compound or an unionized compound into an individual's body surface targeting either local and systemic results, comprising the steps of
(a) using in an area of wetted skin a mild abrasion of the skin surface via the scrubbing effect of ultrasound mechanical vibrations through a special applicator;
(b) contacting said area of the skin with a composition comprising an effective amount of said permeant along with compounds to be permeated;
(c) enhancing the permeability of the selected area to the permeant by means of sonophoresis and applying simultaneously to said area iontophoresis, electroporation, mechanical vibrations and magnetophoresis for a time and with physical properties effective to enhance the transcutaneous flux rate into the body; the improvement comprising: initial skin scrubbing effect via ultrasound mechanical vibrations, simultaneous application of an active permeant and compounds along with sonophoresis, iontophoresis, electroporation, mechanical vibrations and magnetophoresis to an individual's body surface area for a time and with physical properties effective to enhance the transcutaneous flux rate into the body, considerably lowering the required time of application and magnitude of physical parameters compared to those required if the chemical enhancers and compounds to be permeated were used alone;

2. The method of claim 1 wherein a chemical permeation enhancer is also applied to the surface of the area under treatment.

3. The method of claim 1 wherein the ultrasound is a modulated continuous wave.

4. The method of claim 1 wherein the ultrasound is amplitude modulated.

5. The method of claim 1 wherein the modulated ultrasound is a pulsed wave with fixed duty cycle.

6. The method of claim 1 wherein the modulated ultrasound is a pulsed wave with time varying duty cycle.

7. The method of claim 1 wherein the ultrasound is modulated by a combination of different modulation processes.

8. The method of claim 1 wherein the ultrasound is a non-modulated continuous wave.

9. The method of claim 1 wherein the ultrasound has a frequency in the range of about 20 KHz to 10 MHz.

10. The method of claim 1 wherein the ultrasound is a pulsed wave with fixed duty cycle.

11. The method of claim 1 wherein the ultrasound is a pulsed wave with time varying duty cycle.

12. The method of claim 1 wherein the iontophoresis uses an electric field ranging from 0.1 to 25 V.

13. The method of claim 1 wherein the iontophoresis uses a time varying electric field.

14. The method of claim 1 wherein the iontophoresis uses some kind of modulation.

15. The method of claim 1 wherein the iontophoresis uses amplitude modulation.

16. The method of claim 1 wherein the iontophoresis uses frequency modulation.

17. The method of claim 1 wherein the iontophoresis uses duty cycle modulation.

18. The method of claim 1 wherein the iontophoresis uses a combination of electrical modulations.

19. The method of claim 1 wherein either iontophoresis or electroporation are used.

20. The method of claim 1 wherein the mechanical vibrations have constant frequency.

21. The method of claim 1 wherein the mechanical vibrations have frequencies ranging from 1 Hz to about 20 KHz.

22. The method of claim 1 wherein the mechanical vibrations have time varying frequency.

23. The method of claim 1 wherein the heat generated is controlled in such way to keep the skin temperature below 41.degree. C.

24. The method of claim 1 wherein the use of magnetophoresis is optional.

25. The method of claim 1 wherein the magnetic field is constant.

26. The method of claim 1 wherein the magnetic field is time varying.

27. The method of claim 1 wherein sonophoresis is supressed.

28. The method of claim 1 which further comprises using a plurality of transducers for obtaining mechanical scrubbing of the skin and applying other mentioned physical principles, each transducer presenting equal of different physical properties.

29. The method of claim 1 wherein time of application is in the range of about 1 second to 40 minutes.
